# EUROPEAN PATENT APPLICATION

(11) **EP 1 180 687 A1**
(43) Date of publication of application: **20.02.2002**
(21) Application number: 00906380.1
(22) Date of filing: 25.02.2000
(51) Int. Cl.: G01N 33/68, C07K 16/28

(54) **METHOD AND IN VITRO KIT FOR SELECTING A DRUG FOR CHEMOTHERAPY**

(71) Applicant: Balagué Center, S.A., 08908 Hospitalet de Llobregat (ES)
(72) Inventor: PASTOR ANGLADA, Marçal, 08028 Barcelona (ES); FELIPE CAMPO, Antonio, 08028 Barcelona (ES); CASADO MEREDIZ, Francisco Javier, 08028 Barcelona (ES)
(74) Representative: SUGRANES - VERDONCES - FERREGÜELA
(86) International application number: ES0000070
(87) International publication number: WO0052477

(57) **Abstract**

In vitro method and kit for selection of a drug for chemotherapy

Useful for predicting the most suitable nucleoside-related drug to be used in chemotherapy by: (a) contacting a tissue sample of said patient with a series of monoclonal or polyclonal antibodies (e.g. anti hCNT1, anti hCNT2, anti hENT1 and anti hENT2), each one recognizing a specific epitope of a human nucleoside transporter; (b) qualitatively or quantitatively establishing the patient's pattern of nucleoside transporter expression in said sample; (c) comparing said patient's pattern with a set of reference patterns that are known to confer either drug resistance or drug sensitivity; and (d) predicting which one or ones of the considered drugs will be the most appropriate. Preferably biopsies of the patient are processed for immuno-cytochemistry assay, and the establishment of the patient's pattern is done by simple ocular inspection. This is useful in the treatment of cancer.

SEQUENCE LISTING

<110> UNIVERSIDAD DE BARCELONA

<120> METODO Y KIT IN VITRO PARA LA SELECCIÓN DE UN FÁRMACO
      PARA QUIMIOTERAPIA

<130> PASTOR1 WO SPANISH

<140>
<141>

<150> P9900488
<151> 1999-03-01

<160> 3

<170> PatentIn Ver. 2.1

<210> 1
<211> 15
<212> PRT
<213> Homo sapiens

<400> 1
Pro Glu Ser Glu Pro Asp Glu Pro Gln Lys Pro Gly Lys Pro Ser
  1               5                  10                  15

<210> 2
<211> 22
<212> PRT
<213> Homo sapiens

<400> 2
Arg Ser Ser Trp Ser Glu Ala Ala Pro Lys Pro Phe Ser Arg Trp Arg
  1               5                  10                  15

Asn Leu Gln Pro Ala Leu
             20

<210> 3
<211> 11
<212> PRT
<213> Homo sapiens

<400> 3

Ser Asn Ser Gln Pro Thr Asn Glu Ser His Ser
1               5                   10

## Description

This invention relates to human nucleoside transporters and to methods for identification of nucleoside transport proteins, as a tool for molecular diagnosis to predict the sensitivity to a nucleoside related drug.

### BACKGROUND OF THE INVENTION

A general problem of chemotherapy is the difficulty in foreseeing which of the available drugs would have the highest therapeutic index for the treatment of a particular illness in a given patient. In practice many patients present particular resistance to a given drug for not well understood reasons. A method of prediction of the ability of the drug to exert its citotoxic effects would improve the prognosis of the illness and avoid unnecessary treatments.

In chemotherapy some nucleoside related drugs are currently used: fluorouracil, cytarabine, fludarabine, cladribine, gemcitabine, floxidine, dideoxyinosine, dideoxycytidine, dideoxyadenosine, zidovudine, zalcitabine, bromodeoxyuridine, iododeoxyuridine, fluorodeoxyuridine, stavudine, lamivudine and capecitabine, which are used as anticancer and antiviral agents, and others are used in the treatment of cardiovascular diseases. Recently, several cDNAs encoding a variety of nucleoside transporter proteins have been cloned and shown to be responsible for the transport into the cell, not only of naturally occurring nucleosides but also of nucleoside-related drugs used in the chemotherapy of several diseases (cf. M. Pastor-Anglada et al., Trends Pharmacol.Sci. 1998, vol. 19, pp. 424-430). Nucleoside transport into mammalian cells is mediated by concentrative and equilibrative transport systems.

Concentrative transport is dependent on the sodium transmembrane gradient and allows the cell to accumulate the nucleoside against a putative concentration gradient. So far five transport systems have been kinetically identified (N1, N2, N3, N4 and N5). The rat and human counterparts of one N1- and one N2-related cDNAs have been cloned and called rCNT2/hCNT2 (formerly named SPNT) and rCNT1/hCNT1, respectively. CNT refers to Concentrative Nucleoside Transporter, and numbers 1 and 2 refer to the chronological order of publication of the sequences. Thus, CNT2 encodes a N1 type transporter which shows preference for purine nucleosides, and CNT1 encodes a N2 type transporter which shows preference for pyrimidine nucleosides. Prefixes "r" or an "h" refer to the rat and human counterparts of the cDNA, respectively. CNT1 and CNT2 proteins, although encoded by different genes, show significant homology and are considered to belong to the same gene family and thus, are considered to be protein isoforms. So far no cDNA is available that encodes proteins responsible for N3, N4 or N5 transport activities. It is also possible that new isoforms encoding N1 and N2 type transporters remain to be cloned. The pharmacological properties of N3, N4 and N5 are poorly defined, whereas CNT1 has been recently shown to be a suitable transporter for fluoropyrimidines (e.g. gemcitabine), whereas CNT2 does not appear to be a major route for their transport into the cell (cf. J.R. Mackey et al., Cancer Res. 1998, vol. 58, pp. 4349-57).

Equilibrative nucleoside transport refers to the sodium-independent uptake of nucleosides across membranes which is triggered by the transmembrane nucleoside concentration gradient. Two transport systems, es and ei, have been described so far. Both of them show relatively wide substrate specificity, although the former is inhibited by nanomolar concentrations of the nucleoside analog nitrobenzylthioinosine (also known as NBTI or NBMPR). The so-called ei activity is insensitive to NBTI inhibition, even in the micromolar range. The rat and human counterparts of one es-related cDNA (ENT1) and one ei-related cDNA (ENT2) have been cloned so far. ENT refers to Equilibrative Nucleoside Transporter and the numbers refer to the chronological order of publication of the sequences. ENT1 and ENT2 are two isoforms belonging to the same gene family. Other es-and ei-related cDNAs may be cloned in the near future. Thus, CNT and ENT represent two different gene families.

Nucleoside analogs used in chemical and antiviral therapies involve essentially, but not uniquely, purine derivatives (adenosine-derivatives mostly), thymidine analogs and fluoropyrimidines. To exert their pharmacological action they have to become available intracellularly and thus, they must first cross the plasma membrane. Previous evidence and unpublished data demonstrate that these drugs will not be equally accepted by CNT and ENT carriers and thus, the expression pattern of carrier isoforms may be a key determinant of drug bioavailability.

Before the cloning of the first nucleoside transporters there was not a reliable method to analyze carrier expression in human biopsies. Indeed, the only approach available was the determination of specific labelled NBTI binding, which could be understood as an indicator of es-type transporters. NBTI binding is increased in a variety of human tumors and the number of NBTI-binding sites has been correlated in leukaemia cells with cytarabine toxicity. Cytarabine (arabinosylcytosine) is a non-fluorinated pyrimidine derivative effective in the treatment of haematopoietic malignancies. In other cases, the therapeutic efficiency of a nucleoside-related drug can be either potentiated or diminished when inhibiting the equilibrative transport systems, which means there must be a complex relationship between ENT and CNT transporters that determines drug bioavailability. When CNT and ENT cDNA probes have been available, direct demonstration of altered expression of CNT and ENT mRNA levels in tumor cells and their normal counterparts has been provided.

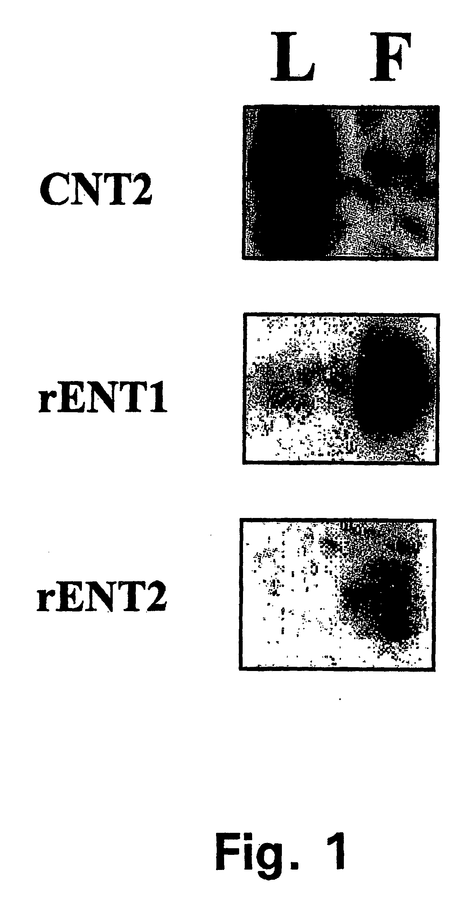

Accompanying Figure 1, reproduced from a published paper from the inventors' laboratory (B. del Santo et al., Hepatology 1998, vol. 28, pp. 1504-1511), shows the steady state levels of rCNT2, rENT1 and rENT2 mRNA in a rat hepatoma cell line and in normal liver parenchymal cells. However, experimental evidence also suggests that mRNA amounts do not necessarily correlate with protein expression, and indeed in liver parenchymal cells a very low amount of rCNT1 is associated with a high level of rCNT1 protein (cf. A. Felipe et al., Biochem.J. 1998, vol. 330, pp. 997-1001). Thus, approaches derived from the previous art, which all involve cDNA probes (Northern blot analysis or in situ hybridization), do not appear to be suitable for solving the problem of quantitation of nucleoside carriers as a tool for the molecular diagnosis of tumor sensitivity to a nucleoside related drug.

### SUMMARY OF THE INVENTION

The present invention provides an in vitro method for predicting the most suitable nucleoside-related drug/s, for prophylaxis or treatment of a given (human) patient suffering from an illness sensitive to said drugs, comprising the steps of:
(a) contacting a tissue sample or extract of said patient with a series of monoclonal or polyclonal antibodies selected from the set of antibodies available at the time of prediction, each one recognizing a specific epitope of a human nucleoside transporter, for a time and under conditions sufficient to form antigen-antibody complexes and detecting the formation of the complexes;
(b) qualitatively or quantitatively establishing the patient's pattern of nucleoside transporter expression in said sample, for the selected antibodies;
(c) comparing said patient's pattern with a set of reference patterns that, at the time of prediction, are known to confer either drug resistance or drug sensitivity in said illness, for the set of drugs considered; and
(d) predicting which one or ones of the considered drugs will be the most appropriate for prophylaxis or treatment of said patient in said illness.

In a preferred embodiment, the detection of the complex formation in step (a) is carried out through a technique selected from the group consisting of radioimmuno assay, immuno-enzymatic assay and immunofluorescent assay, the most preferred ones being immuno-enzymatic assay and immunofluorescent assay. Particularly suitable is the technique of immuno-cytochemistry assay on a biopsy.

In a preferred embodiment, in the above-mentioned diagnosis method the establishment of the patient's pattern in step (b) is done qualitatively by simple ocular inspection.

When this invention was made only anti hENT1 had been mentioned (although not described) as antibody against human nucleoside transporters. Here new polyclonal antibodies anti hENT1, anti hENT2 and anti hCNT1 are described. Thus, in a preferred embodiment, the method of the invention involves a series of antibodies comprising anti hCNT1, anti hCNT2, anti hENT1 and anti hENT2. As soon as other antibodies of this type become available, the series will be increased or changed accordingly.

When this invention was made, the list of available nucleoside-related drugs used in chemotherapy included, among others, the following: fluorouracil, cytarabine, fludarabine, cladribine, gemcitabine, floxidine, dideoxyinosine, dideoxycytidine, dideoxyadenosine, zidovudine, zalcitabine, bromodeoxyuridine, iododeoxyuridine, fluorodeoxyuridine, stavudine, lamivudine and capecitabine. Other compounds will be added to the list above, as soon as new drugs become available, or as a result of research or development of new drugs.

Another aspect of the present invention relates to an in vitro diagnosis kit for the prediction of the most suitable nucleoside-related drug/s, for prophylaxis or treatment of a given patient suffering from an illness sensitive to said drug/s, comprising a series of monoclonal or polyclonal antibodies selected from the set of antibodies available at the time of prediction, each one recognizing a specific epitope of a human nucleoside transporter.

In a preferred embodiment, the diagnosis kit further comprises information about a set of reference patterns that, at the time of prediction, indicate which drug or drugs are believed to be the most suitable for a given pattern of nucleoside transporter expression. In another preferred embodiment the kit provides a protocol for qualitatively or quantitatively establishing the patient's pattern of nucleoside transporter expression in a tissue sample or extract of a patient suffering from an illness sensitive to nucleoside-related drugs. And, in general, the diagnosis kit can also comprise any material or information needed for carrying out any of the above-mentioned diagnosis methods.

As illustrated in the accompanying examples, the method of the present invention can use polyclonal antibodies against CNT isoforms as tools to analyze carrier expression in tumor biopsies (in the examples anti rCNT1 and anti rCNT2 were used, but in human practice anti hCNT1 and anti hCNT2 are used). In fact, the examples of the present invention are the first illustration of the utilization of antibodies against nucleoside carriers for molecular diagnosis, by providing data using antipeptide antibodies against the CNT1 and CNT2 isoforms in biopsies from liver tumors.

The rationale for the invention is that, once established the substrate specificity pattern of the available isoforms (those cloned at every renewal of the invented kit), the analysis of the carrier isoform expression pattern of a tumor biopsy, performed, ideally, but not uniquely using an immuno-cytochemistry approach, will provide a know how to proceed regarding the most suitable nucleoside-related drug to be used for chemotherapeutical purposes. In cases when biopsies from patients undergoing antiviral therapies become available because of their particular pathophysiological conditions, the analysis of the expression pattern of nucleoside transporters in a certain tissue may be also useful for molecular diagnosis and prediction of drug sensitivity.

The same type of analysis will also be possible for the ENT isoforms and any other not-yet cloned isoform, whenever monoclonal or polyclonal antibodies become available. When this invention was made one policlonal antibody anti hENT1 had been mentioned, but not describer (cf. M. Griffiths et al., Nature Medicine 1997, vol. 3, pp. 89-93). Here the first policlonal antibodies anti hENT2 and anti hCNT1 are described, as well as a new anti hENT1.

Thus, in a particular embodiment this invention provides a new polyclonal antibody anti hENT2, against the human equilibrative nucleoside transporter, which is obtained by standard immunization with a protein conjugated with the peptide of SEQ ID NO 1, or with a peptide with a sequence resultant from adding, to SEQ ID NO 1, the amino acid residues necessary for protein conjugation. In a preferred embodiment the conjugated protein is the keyhole limpet hemocyanin.

In another particular embodiment, a new polyclonal antibody anti hCNT1 is provided, against the human concentrative nucleoside transporter, which is obtained by standard immunization with a protein conjugated with the peptide of SEQ ID NO 2, or with a peptide with a sequence resultant from adding, to SEQ ID NO 2, the amino acid residues necessary for protein conjugation. In a preferred embodiment the conjugated protein is the keyhole limpet hemocyanin.

In another embodiment, a new polyclonal antibody anti hENT1 is provided, against the human equilibrative nucleoside transporter, which is obtained by standard immunization with a protein conjugated with the peptide of SEQ ID NO 3, or with a peptide with a sequence resultant from adding, to SEQ ID NO 3, the amino acid residues necessary for protein conjugation. In a preferred embodiment the conjugated protein is the keyhole limpet hemocyanin.

### BRIEF DESCRIPTION OF THE DRAWINGS

Figure 1 (cf. Del Santo et al., ibid.) illustrates the mRNA levels of the nucleoside transporter isoforms rCNT2, rENT1 and rENT2 in rat liver parenchymal cells (L), and the rat hepatoma cell line FAO (F).
Figure 2 shows the Western blot analysis of rCNT1 and rCNT2 proteins in biopsies from normal rat liver (L), from chemically-induced tumors (C), and from transgenic tumors (T).
Figure 3 shows histochemical staining and immunostaining of serial tissue sections from the liver of a Solt-Farber treated rat containing an adenoma. (HE) means hematoxylin and eosin; (GT) means glutathione-S-transferase; (GP) means glucose-6-phosphatase; and (rCNT2) means concentrative nucleoside transporter 2.
Figure 4 shows histochemical staining and immunostaining of serial tissue sections from the liver of a transgenic rat with an hepatocellular carcinoma. (HE) means hematoxylin and eosin; (AT) means T-antigen; (GP) means glucose-6-phosphatase; and (rCNT1) means concentrative nucleoside transporter 1.

### DESCRIPTION OF PREFERRED EMBODIMENTS

The present invention is illustrated by the following examples.

### EXAMPLE 1: Materials and methods

### 1.1) Rat models of hepatocarcinogenesis

The expression of the two rCNT isoforms present in liver parenchymal cells was studied in two models of hepatocarcinogenesis: Solt and Farber model (D. Solt and E. Farber, Nature 1976, vol. 263, pp. 701-3), and albumin-SV40 large T antigen (Alb-SV40) transgenic rat (J.R. Hully et al., Am. J. Pathol. 1994, vol. 145, pp. 384-97). Rats bearing hepatocarcinomas were killed and livers were either dissected, to isolate the individual tumors from surrounding normal tissue, or wholly frozen in liquid nitrogen, or sectioned and frozen as a block on solid carbon dioxide for further histochemical or immunohistochemical analysis. Serial sections of 10-micron thickness were obtained.

### 1.2) Anti-rCNT1 and anti-rCNT2 antibodies. Western blot analysis.

Isoform-specific anti-rCNT1 and anti-rCNT2 antibodies were obtained from rabbits injected with KLH-coupled oligopeptides, corresponding to amino acid residues 45-67 and 30-52 for rCNT1 and rCNT2 respectively. Liver tissue was homogenized in a buffer consisting of 0.5% Triton X-100 10 mM Tris (pH 7.4) with an Ultra-Turrax® polytron (from Ika). Liver homogenates were used for protein determination prior to SDS-PAGE. Twenty-microgram protein samples were then heated for 6 min at 1000C and run on a 10% polyacrylamide gel. Proteins were transferred onto filters (Immobilon-P® from Millipore), and these were blocked in a 5% dry milk-supplemented, 0.2% Tween-20 Tris buffer saline (TBS) solution prior to immunoreaction. Filters were then incubated for 1h with the diluted antisera (1/500 in a 0.2% Tween-20 TBS solution supplemented with 1% bovine serum albumin). A goat anti-rabbit IgG, coupled to horseradish peroxidase, was used as a second antibody and diluted 1/2000 in dry milk-supplemented 0.2% Tween-20 TBS. To visualize the CNT1 and CNT2 proteins, the ECL technique (from Boehringer) was used.

### 1.3) Immunolocalization of rCNT1 and rCNT2 proteins

The antibodies against rCNT1 and rCNT2 proteins were used for immuno-cytochemistry. Cryosections of liver (6-8 mm) were thaw-mounted onto 3-aminopropyltriethoxysilane-coated slides, air dried, and then fixed in 4% p-formaldehyde for 30s. Slides were washed three times in PBS for 5 min and then endogenous peroxidase activity was blocked by incubating for 30 min in 0.3% H₂O₂ in methanol. Slides were washed again in PBS, and endogenous biotin was blocked with 50 mg/ml avidin. After further washing in PBS, the sections were incubated for 30 min at room temperature in NBS (Normal Blocking Serum), which consisted of 5% Normal Goat Serum and 1% BSA. This step was applied to decrease the background from nonspecific protein binding and to enhance sensitivity. These antibodies were diluted from 1/500 to 1/3000 in NBS and incubated with the sections overnight at 40 C. The slides were washed three times in PBS and then incubated for 30 min at room temperature with the biotinylated secondary antibody (Anti-rabbit IgG Biotin Conjugate, from Sigma, St. Louis, MO, USA) diluted 1/2000 in NBS. The antigen-antibody complexes were visualized by the biotin-streptavidin-peroxidase method with aminoethyl carbazole as the chromogen. The sections were counterstained with Mayer's hematoxylin to permit visualization of the nuclei, and the sections were coverslipped with Crystal mount (from Biomeda, Foster City, CA, USA).

### EXAMPLE 2: Use of Western blot analysis to monitor rCNT carrier isoform expression

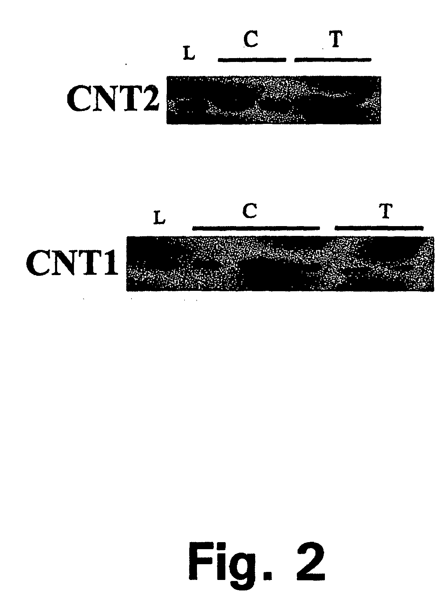

Western blot analysis of liver samples from transgenic rats developing hepatocarcinogenesis showed that cancer is associated with a significant decrease in rCNT1 protein levels (cf. Figure 2). Indeed, these samples were contaminated by normal tissue and this type of analysis may not be suitable for diagnosis (as shown in the case of rCNT2 expression in chemically induced tumors, where no apparent changes in protein levels were detected).

### EXAMPLE 3: Use of immuno-cytochemistry to monitor rCNT carrier isoform expression

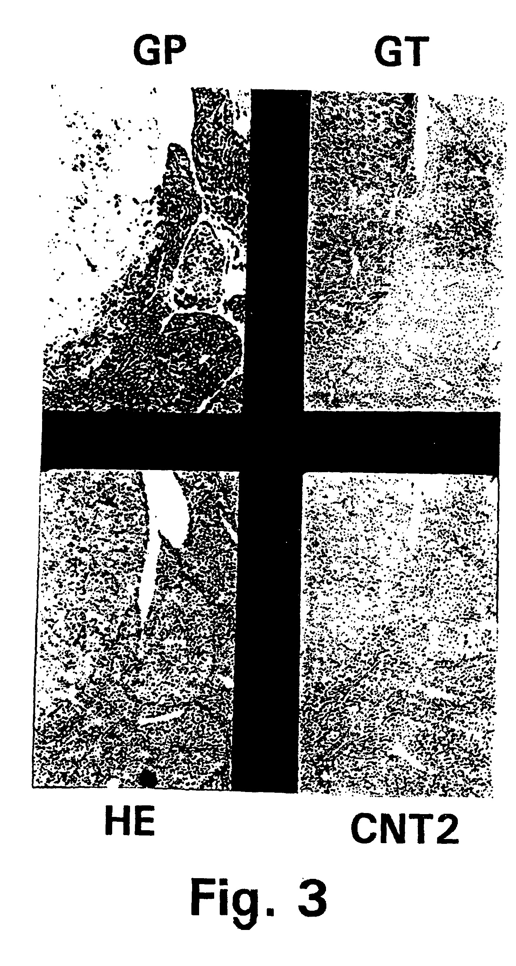

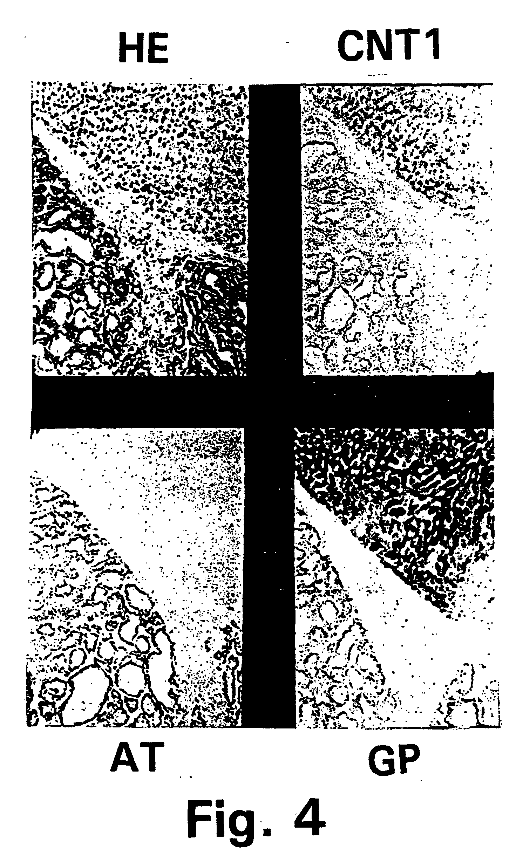

The results of this example, illustrated in Figures 3 and 4, show that the rCNT1 isoform was not expressed in tumors from transgenic rats (Fig. 4), whereas rCNT2 expression was decreased in chemically-induced tumors (Fig. 3).

### EXAMPLE 4: Reference pattern of liver tumors and prediction of sensitivity to gemcitabine

It is known that gemcitabine uptake is mostly mediated by (rat and human) CNT1, this drug being a poor substrate for (rat and human) CNT2 (J.R. Mackey et al., ibid). In Figure 4, HE panel shows a normal parenchyma (granular part) and a glandular tumor (rugged part). GP Panel shows that a liver-specific enzyme is expressed in normal parenchyma but not in the tumor. rCNT1 panel shows the same distribution for the rCNT1 nucleoside transporter, which means that the liver tumors in the studied transgenic animals do not express rCNT1. From this information it is predicted that treatment with gemcitabine will not be suitable for that specific tumor.

In Figure 3, HE panel shows two tumor nodules, which are further identified by their dark staining in the GT panel. GP panel also shows that the same liver-specific enzyme marker is poorly expressed in the tumor nodules. However, rCNT2 panel shows that the rCNT2 isoform is poorly expressed in this particular biopsy (whereas rCNT1 is expressed normally; results not shown). From this information it is predicted that treatment with gemcitabine may be suitable for that specific tumor.

A generalization of this kind of information could be presented in a reference pattern with the design of Table 1. In such a pattern, sensitivity to gemcitabine would be consistent with hCNT1 (+) and hCNT2 (irrel.). Behavior of hENT1 and hENT2 transporters is to be determined.

**Table 1:**

| Example of a general reference pattern for prediction (+/- means transporter presence/absence; irrel. means irrelevant as decision criteria). | | | | | |
|---|---|---|---|---|---|
| | hCNT1 | hCNT2 | hENT1 | hENT2 | OTHERS |
| (no treatment) | - | - | - | - | - |
| DRUG1 | + | - | irrel. | - | ... |
| DRUG2 | - | + | + | - | ... |
| DRUG3 | + | irrel. | + | irrel. | ... |
| OTHERS | ... | ... | ... | ... | ... |

## Claims

1. An in vitro method for predicting the most suitable nucleoside-related drug or drugs, for prophylaxis and/or treatment of a given (human) patient suffering from an illness sensitive to said drug/s, comprising the steps of:
(a) contacting a tissue sample or extract of said patient with a series of monoclonal or polyclonal antibodies, each one recognizing a specific epitope of a human nucleoside transporter, for a time and under conditions sufficient to form antigen-antibody complexes and detecting the formation of the complexes;
(b) qualitatively or quantitatively establishing the patient's pattern of nucleoside transporter expression in said sample or extract, for the selected series of antibodies;
(c) comparing said patient's pattern with a set of reference patterns that, at the time of prediction, are known to confer either drug resistance or drug sensitivity in said illness, for the set of drugs considered; and
(d) predicting which one or ones of the considered drugs will be the most appropriate for prophylaxis or treatment of said patient in said illness.

2. A method according to claim 1, wherein the detection of the complex formation in step (a) is carried out through a technique selected from the group consisting of radioimmuno assay, immuno-enzymatic assay and immunofluorescent assay.

3. A method according to claim 2, wherein the detection technique is immuno-enzymatic assay.

4. A method according to claim 2, wherein the detection technique is immunofluorescent assay.

5. A method according to any of the claims 3 and 4, wherein a tissue sample of the patient is a biopsy, and this is processed for immuno-cytochemistry assay.

6. A method according to any of the preceding claims, wherein the establishment of the patient's pattern in step (b) is done qualitatively by simple ocular inspection.

7. A method according to any of the preceding claims, wherein the series of available antibodies comprises the following polyclonal antibodies: anti hCNT1, anti hCNT2, anti hENT1 and anti hENT2.

8. A method according to any of the preceding claims, wherein the drugs of the reference pattern in step (c) comprises a set selected from the group consisting of fluorouracil, cytarabine, fludarabine, cladribine, gemcitabine, floxidine, dideoxyinosine, dideoxycytidine, dideoxyadenosine, zidovudine, zalcitabine, bromodeoxyuridine, iododeoxyuridine, fluorodeoxyuridine, stavudine, lamivudine and capecitabine.

9. An in vitro kit for the prediction of the most suitable nucleoside-related drug or drugs, for prophylaxis and/or treatment of a given patient suffering from an illness sensitive to said drug/s, comprising a series of monoclonal or polyclonal antibodies, each one recognizing a specific epitope of a human nucleoside transporter.

10. A kit according to claim 9, further comprising information about a set of reference patterns that, at the time of prediction, indicates which drug or drugs are believed to be the most suitable for a given pattern of human nucleoside transporter expression.

11. A kit according to claim 9, further comprising a protocol for qualitatively or quantitatively establishing the patient's pattern of human nucleoside transporter expression in a tissue sample or extract of a patient suffering from an illness sensitive to nucleoside-related drugs.

12. A kit according to claim 9, comprising the materials needed for carrying out any of the in vitro diagnosis methods defined in any of the claims 2-8.

13. A polyclonal antibody anti hENT2, against the human equilibrative nucleoside transporter, obtainable by immunization with a protein conjugated with the peptide of SEQ ID NO 1, or with a peptide with a sequence resultant from adding, to SEQ ID NO 1, the amino acid residues necessary for protein conjugation.

14. A polyclonal antibody anti hCNT1, against the human concentrative nucleoside transporter, obtainable by immunization with a protein conjugated with the peptide of SEQ ID NO 2, or with a peptide with a sequence resultant from adding, to SEQ ID NO 2, the amino acid residues necessary for protein conjugation.

15. A polyclonal antibody anti hENT1, against the human equilibrative nucleoside transporter, obtainable by immunization with a protein conjugated with the peptide of SEQ ID NO 3, or with a peptide with a sequence resultant from adding, to SEQ ID NO 3, the amino acid residues necessary for protein conjugation.

16. A polyclonal antibody according to any of the claims 13-15, wherein the conjugated protein is the keyhole limpet hemocyanin.

17. A polyclonal antibody according to any of the claims 13-16, wherein the said immunization is carried out with rabbits.
